# FASCICULE DE BREVET EUROPEEN

(11) **EP 3 002 142 B1**
(45) Date de publication et mention de la délivrance du brevet: **29.08.2018**
(21) Numéro de dépôt: 15185129.2
(22) Date de dépôt: 14.09.2015
(51) Int. Cl.: B60H 3/00, B60R 25/00, B60R 25/21, A61L 9/12

(54) **SYSTÈME DE VERROUILLAGE DE CARTOUCHE AMOVIBLE DANS UN BOÎTIER**
VERRIEGELUNGSSYSTEM EINER ENTFERNBAREN PATRONE IN EINEM GEHÄUSE
SYSTEM FOR LOCKING A REMOVABLE CARTRIDGE IN A HOUSING

(30) Priorité: 30.09.2014 FR 1459329
(43) Date de publication de la demande: 06.04.2016
(73) Titulaire: VALEO SYSTEMES THERMIQUES, 78320 Le Mesnil Saint-Denis (FR)
(72) Inventeur: QUEINNEC, Jean-Yves, 78990 ELANCOURT (FR); LECHAT, Yvan, 78120 SONCHAMP (FR)
(74) Mandataire: Metz, Gaëlle

(56) Documents cités:
- WO-A2-2009/003704
- DE-U1-202004 010 015
- FR-A1- 2 918 318
- US-A1- 2004 016 818
- US-A1- 2004 028 449
- US-A1- 2012 286 633

## Description

La présente invention concerne un système de verrouillage de cartouche amovible dans un boîtier, en particulier destiné à être utilisé dans un dispositif de diffusion faisant partie d'un véhicule. Un tel système de verrouillage est connu, par exemple, du document FR2918318, qui concerne un dispositif de diffusion faisant partie d'un véhicule automobile. Il est connu des dispositifs comprenant un boîtier et pourvus d'une cartouche amovible, et d'un bouton de verrouillage dudit élément amovible par rapport au boîtier.

C'est le cas des dispositifs de diffusion que l'on trouve dans certains véhicules automobiles. Des agents volatils sont logés dans la cartouche qu'il est nécessaire de changer lorsque les agents volatils sont épuisés. Le bouton de verrouillage/déverrouillage placé sur le boîtier est accessible au passager. Il permet l'enlèvement de la cartouche. Cependant, avant la livraison du véhicule automobile, en particulier pendant son transport au concessionnaire, la cartouche amovible est déjà disposée dans le dispositif de diffusion.

Il peut être tentant, avant la livraison du véhicule, à n'importe quel individu de prendre cette cartouche amovible qui est un élément coûteux du véhicule.

Afin d'éviter d'éventuels vols, il serait avantageux de disposer d'un système de blocage de ladite cartouche amovible qui ne permette pas son enlèvement du boîtier.

Il existe dès lors un besoin de disposer d'un système de verrouillage efficace avant la livraison du véhicule, qui puisse être simplement débloqué lorsque le véhicule est vendu.

L'invention propose un système de verrouillage de cartouche amovible dans un boîtier, ledit système comprenant un organe mobile, destiné à fermer et/ou ouvrir une et/ou des ouvertures de ladite cartouche, ledit système comprenant en outre un déclencheur, destiné à autoriser une extraction de ladite cartouche hors du boîtier en cas d'actionnement, ledit organe mobile étant configuré pour bloquer l'actionnement dudit déclencheur de manière à empêcher à la cartouche de sortir du boîtier, suivant les caractéristiques de la revendication 1. Ainsi, selon l'invention, on empêche l'extraction de la cartouche non pas à l'aide d'un système supplémentaire mais en se servant du déclencheur permettant l'extraction, lui-même, dont on bloque l'actionnement.

Selon différents modes de réalisation de l'invention qui pourront être pris ensemble ou séparément :
- ledit système comprend des moyens de commande de l'organe mobile, configurés pour que l'organe mobile se déplace dans la première plage de déplacement dans un premier mode d'utilisation du système et dans la seconde plage de déplacement dans un second mode d'utilisation du système,
- le plateau est mobile en rotation,
- la butée de blocage empêche en particulier l'actionnement du déclencheur dans la seconde plage de déplacement dudit plateau,
- le système comprend en outre un axe d'actionnement dudit plateau,
- lesdits moyens de transmission comprennent une tige, un axe de rotation et un ressort de rappel,
- lesdits moyens de transmission sont configurés pour que ledit ergot se cale dans une cavité creusée sur la cartouche amovible lorsque la cartouche est insérée dans le boîtier et se relâche lorsque le bouton est actionné, en particulier dans la première plage de déplacement,
- ledit déclencheur est un ressort configuré pour agir sur ladite cartouche amovible et ladite butée est un bord périphérique, angulairement interrompue, dudit plateau,
- ledit bord périphérique du plateau est configuré pour coopérer avec un ergot de ladite cartouche amovible.

L'invention concerne aussi un ensemble comprenant le système de verrouillage évoqué plus haut et une cartouche amovible.

Selon différents modes de réalisation de l'invention qui pourront être pris ensemble ou séparément :
- la cartouche amovible est pourvue d'une cavité,
- la cartouche comprend une pluralité d'ouvertures, en particulier en communication avec les dites cavités, et ledit organe mobile est configuré pour sélectionner l'ouverture ouverte parmi les ouvertures de la cartouche,
- ladite cartouche amovible comprend en outre une fente configurée pour laisser passer ledit axe d'actionnement,
- ladite cartouche amovible comprend un ergot configuré pour coopérer avec ledit bord périphérique du plateau.

L'invention concerne aussi un dispositif de diffusion comprenant un ensemble tel que décrit plus haut.

Selon différents modes de réalisation de l'invention qui pourront être pris ensemble ou séparément :
- lesdites cavités logent au moins un agent volatil,
- les agents volatils comprennent des billes odorantes,
- la cartouche comprend une façade d'introduction dans ledit boîtier,
- les différentes cavités de la cartouche forment des portions angulaires d'un anneau,
- ladite cartouche comprend un corps et un couvercle,
- ledit corps est compartimenté pour définir lesdites cavités,
- ledit couvercle est muni de la cavité coopérant avec l'ergot du système de verrouillage,
- ledit organe mobile forme un sélecteur,
- ledit sélecteur comprend au moins une entrée et une sortie d'un flux d'air, ledit dispositif de diffusion étant configuré pour mettre en communication ladite entrée et ladite sortie, avec l'une au moins desdites cavités pour que cette dernière soit traversée par ledit flux d'air.

L'invention sera mieux comprise à la lumière de la description suivante qui n'est donnée qu'à titre indicatif et qui n'a pas pour but de la limiter, accompagnée des dessins joints parmi lesquels :
- la figure 1 illustre une vue globale en perspective d'un dispositif de diffusion muni d'un système de verrouillage selon un premier mode de mise en oeuvre de l'invention,
- la figure 2 illustre le dispositif de la figure 1 sans son boîtier,
- la figure 3 illustre une vue en perspective de la cartouche du dispositif de la figure 1,
- les figures 4a à 4e illustrent de façon schématique, en vue de dessus, les différentes positions du sélecteur du dispositif de diffusion de la figure 1,
- les figures 5a à 5e illustrent les différentes positions du système de verrouillage/déverrouillage de la figure 1,
- la figure 6 illustre de façon schématique, en vue de dessus, la position de l'organe mobile du système de verrouillage de la figure 1 lorsque l'actionnement de son bouton de verrouillage/déverrouillage est bloqué
- la figure 7 illustre le dispositif de diffusion de la figure 1 avec une variante de mise en oeuvre du système de verrouillage conforme à l'invention.

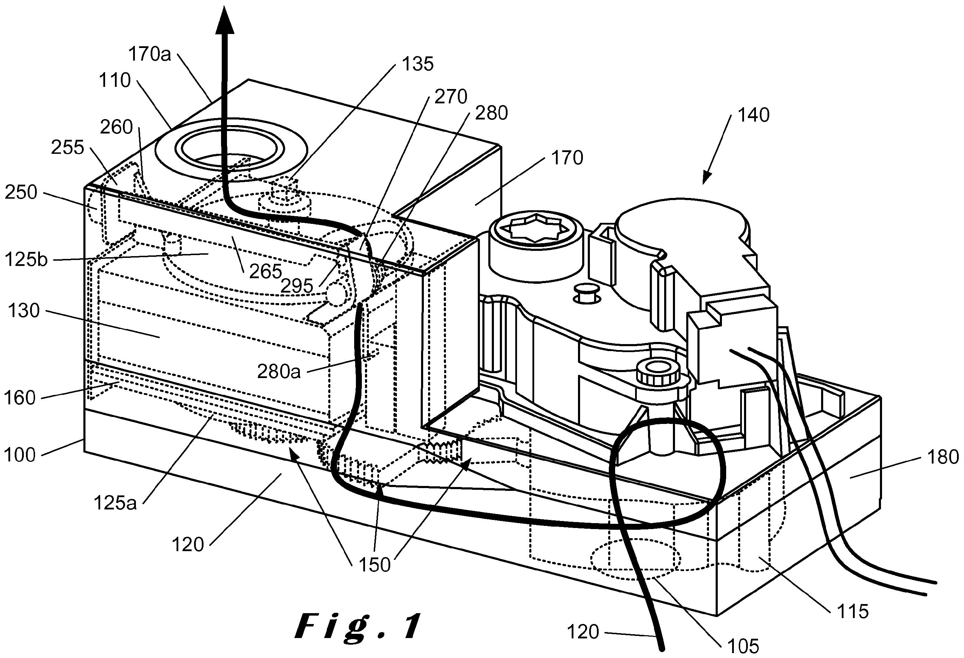

Comme illustré sur la figure 1, l'invention concerne un système de verrouillage de cartouche amovible dans un boîtier, en particulier pour un dispositif de diffusion permettant le traitement d'un flux d'air à l'aide d'agents volatils, notamment de fragrances.

Selon un mode de réalisation particulièrement avantageux de l'invention, les agents volatils, notamment les fragrances, comprennent un substrat, notamment des billes, chargé en composé(s) volatil(s). Il pourra en particulier s'agir de billes odorantes.

Le diffuseur est illustré sur cette figure dans son ensemble. Il comprend ici un boîtier 100 sur lequel deux orifices servent d'entrée 105 et de sortie 110 de l'air. Dès lors, le boîtier sert de guide d'air et comprend une pluralité de composants qui vont permettre à l'air de circuler dans le boîtier. De préférence, une interface sur les orifices 105, 110 permet à une tubulure, non représentée, de s'y fixer tout en assurant l'étanchéité du système à l'air.

Le diffuseur comprend une cartouche 130 de fragrances et un sélecteur, ici logés dans ledit boîtier 100. Le sélecteur comprend, par exemple, un sélecteur d'entrée 125a, un sélecteur de sortie 125b et un axe mobile 135 les entraînant. Ledit diffuseur comprend en outre ici un mécanisme d'actionnement 150, permettant un entraînement dudit axe 135.

Le boîtier 100 peut être fixé à différents emplacements du véhicule, de préférence sur la planche de bord ou dans la boîte à gants afin que ce dernier soit facilement accessible au passager. En effet, la cartouche est amovible par rapport audit boîtier et il est avantageux que le passager puisse facilement changer la cartouche.

La sortie d'air du boîtier 110 est reliée, par exemple, à une grille par laquelle l'air traité ou parfumé sort. Cette grille peut être fixée à différents emplacements dans le véhicule. De préférence, elle est située sur la planche de bord ou près des systèmes de ventilation du pare-brise, la grille étant reliée à la sortie du boîtier par la tubulure déjà évoquée.

Ici, le boîtier 100 comprend une base 180, par exemple parallélépipède rectangle. Dans la base sont logés un pulseur 115 situé au-dessus de l'entrée 105 du boîtier et au moins une partie du mécanisme d'actionnement 150. Ledit mécanisme d'actionnement pourra comprendre un moteur 140. Ce dernier est fixé sur le boîtier 100 au-dessus du pulseur 115, et se trouve en dehors du boîtier. Au-dessus de la base 180, et à côté du moteur 140, une seconde partie du boîtier 170 possède une forme sensiblement cubique pour y loger le sélecteur de fragrances et la cartouche 130. Ces parties du boîtier sont assemblées pour n'en former qu'une seule. L'étanchéité à l'air est assurée dans l'ensemble du boîtier. L'orifice d'entrée 105 est donc dans la base du boîtier 180, sur la face inférieure et en dessous du pulseur 115. L'orifice de sortie 110 est sur la face supérieure de la deuxième partie 170, au-dessus du sélecteur de sortie 125b.

L'air pénètre dans le boîtier par l'orifice d'entrée 105, en étant aspiré par le pulseur 115. Ce pulseur génère le flux d'air qui va finalement sortir du boîtier par l'orifice de sortie 110 après avoir traversé la cartouche 130. Le flux d'air est illustré par la ligne 120 sur la figure 1. De préférence, le pulseur 115 est de type radial, mais il peut également être de type axial.

La cartouche 130 est avantageusement composée d'une pluralité de cavités dans lesquelles différentes fragrances peuvent être logées. Le sélecteur 125a, 125b est configuré de sorte que l'une ou plusieurs de ces cavités soient traversées par le flux d'air afin que celui-ci soit chargé des composés volatils de la ou des fragrances sélectionnées. Autrement dit, lorsque le flux d'air traverse la ou lesdites cavités sélectionnées, l'air vient en contact avec l'ensemble du substrat contenu dans chacune desdites cavités et ledit substrat libère le ou les composés qui vont parfumer et/ou traiter le flux d'air, et ensuite l'habitacle du véhicule automobile.

Pour cela, le flux d'air 120 pénètre dans la cartouche 130 par une entrée du sélecteur d'entrée. Le flux d'air traverse alors la cartouche 130 de part en part en passant par l'une ou plusieurs des cavités de ladite cartouche qu'il quitte à travers une sortie dudit sélecteur de sortie 125b. En faisant traverser la ou les cavités sélectionnées par le flux d'air, on s'assure d'un chargement optimisé du flux d'air par la fragrance, celui-ci venant au contact de l'ensemble de la surface du substrat associé. Après être sortie de la cartouche, le flux d'air est ici guidé jusqu'à l'orifice de sortie 110 du boîtier.

De manière préférentielle, le pulseur 115 permet au dispositif d'être indépendant du système de ventilation, de climatisation ou de chauffage dont le véhicule est éventuellement équipé. Il peut dès lors être utilisé indépendamment de ces systèmes, ce qui le rend d'autant plus flexible à l'utilisation. Dans un autre mode de réalisation, il est relié au système de ventilation, de climatisation ou de chauffage du véhicule. L'orifice d'entrée 105 et de sortie 110 seraient dans ce mode de réalisation relié à un conduit dudit système.

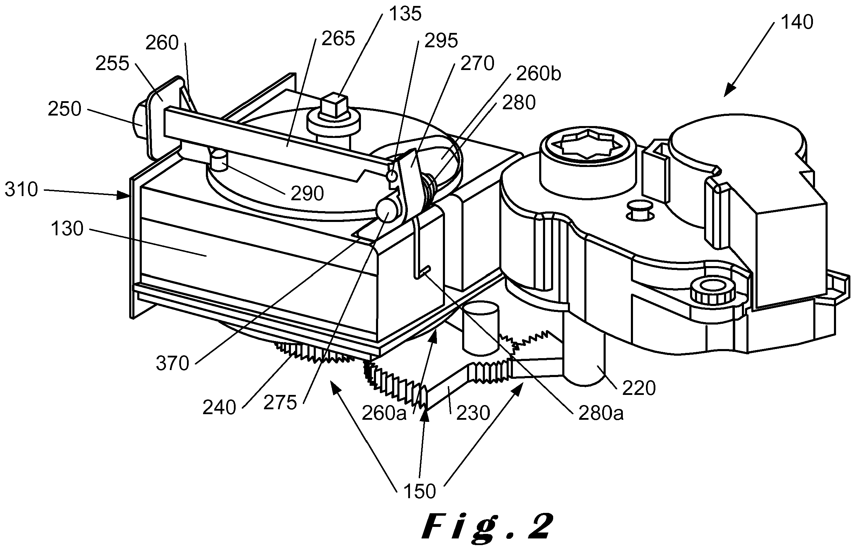

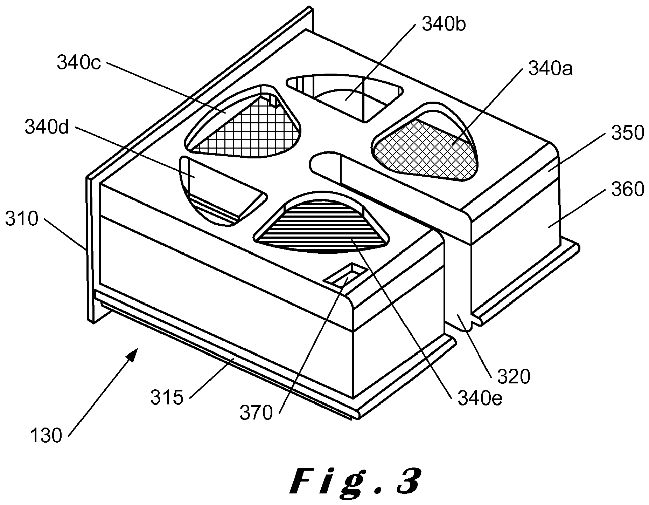

La figure 2 offre une vue plus détaillée de la figure 1 sans le boîtier 100. La figure 2 illustre les détails du mécanisme d'actionnement 150, de l'axe mobile 135 et du sélecteur d'entrée 125a et de sortie 125b. La cartouche 130 est illustrée plus en détails sur la figure 3.

Le mécanisme d'actionnement 150 comprend par exemple le moteur 140, un arbre 220, un pignon de transmission 230, permettant avantageusement une démultiplication, et un pignon droit 240. Le moteur 140 entraîne la cinématique des éléments situés en aval : l'arbre 220, directement relié à un actuateur du moteur, entraîne le pignon de transmission 230, qui entraîne le pignon droit 240. L'axe mobile 135 est directement fixé sur le pignon droit 240 et est dès lors également entraîné par ce dernier.

Avantageusement, le sélecteur d'entrée 125a et le sélecteur de sortie 125b se présentent sous la forme de plateaux tournants, l'axe mobile 135 perçant les deux plateaux tournants en leur centre. La forme du trou perçant les plateaux tournants correspond à la forme d'une section de l'axe mobile, de préférence de forme carrée de telle sorte que lorsque l'axe mobile tourne, les plateaux tournants tournent également. Les plateaux tournants sont séparés par une distance correspondant à la hauteur de la cartouche 130 afin de permettre à la cartouche d'y être insérée. Lorsque la cartouche est insérée entre les deux plateaux, ces derniers sont avantageusement pressés contre la cartouche par des ressorts, non illustrés, afin d'assurer l'étanchéité à l'air.

De préférence, l'entrée et la sortie du flux d'air sur les plateaux tournants situés respectivement en amont et en aval de la cartouche se présentent sous la forme d'ouvertures débouchantes 260b (l'ouverture 260a en amont de la cartouche n'est pas visible) dont la taille est comparable à celle d'une entrée, respectivement d'une sortie d'air, d'une cavité de la cartouche. Les ouvertures des deux plateaux tournants sont disposées en vis-à-vis de manière à assurer la continuité du flux d'air. D'autre part, les positions des ouvertures desdits plateaux tournants restent synchronisées pendant leur mouvement grâce à l'axe mobile 135 qui les relie.

La figure 3 offre une vue de la cartouche isolée 130. Comme déjà dit, la cartouche 130 est un élément amovible par rapport au boîtier 100. Le passager du véhicule peut alors facilement changer de cartouche, notamment lorsqu'elle est vide, la durée de vie d'une cartouche étant d'environ 3 mois pour une utilisation normale. Par « vide », on entend dont les fragrances sont épuisées et ne libèrent plus de composés volatils.

La cartouche est ici munie d'une façade d'introduction 310 dans ledit boîtier, se présentant de manière similaire à la façade d'un tiroir. Cette façade 310 facilite l'insertion de ladite cartouche 130 dans le boîtier 100. La cartouche 130 est également pourvue d'une nervure longitudinale 315 de guidage située le long des trois faces verticales qui vont s'insérer dans le boîtier 100. Pour accueillir lesdites nervures de guidage 315 de la cartouche 130, le boîtier possède également des rainures de guidage 160, illustrées sur la figure 1, de part et d'autre du logement de la cartouche dans le boîtier et au-dessus du plateau tournant inférieur 125a.

Une fente 320 permet le passage de l'axe lorsque la cartouche est insérée dans le boîtier entre les deux plateaux tournants 125a et 125b illustrés aux figures 1 et 2. En position d'utilisation, l'axe mobile 135 se trouve au centre de la cartouche dans le fond de la fente.

La cartouche comprend un corps 360 et un couvercle 350 dont la forme est parallélépipédique et la base est sensiblement carrée. Le corps est compartimenté pour définir lesdites cavités, le fond dudit corps comprend les entrées du flux d'air desdites cavités, et le couvercle comprend les sorties du flux d'air desdites cavités. La cartouche est de préférence en matière plastique. Lesdites ouvertures pourront être revêtues d'un grillage ou d'un film poreux pour retenir le substrat desdites fragrances tout en laissant passer le flux d'air à travers.

Avantageusement, des cavités occupées par une fragrance et des cavités vides alternent. L'alternance de cavités vides et de cavités logeant une fragrance permet de passer de chacune des cavités comprenant un fragrance à une cavité n'en comportant pas, sans passer par un cavité comprenant une autre fragrance.

Dans un mode de réalisation tel qu'illustré sur la figure 3, les différentes cavités de la cartouche et leurs ouvertures respectives forment des portions angulaires d'un anneau. Dans ce mode de réalisation particulièrement avantageux, cinq cavités sont prévues, dont trois pour loger une fragrance 340a, 340c, 340e et deux sont laissées vides intentionnellement 340b, 340d. L'angle balayé par les ouvertures de chaque portion angulaire est dans ce mode de réalisation légèrement inférieur à 60 degrés. Dans un tel mode de réalisation, les ouvertures 260a, 260b des plateaux se présentent également sous la forme d'une portion angulaire, chaque plateau comprenant ici une unique ouverture 260a, respectivement 260b.

Le passager peut dès lors choisir entre trois fragrances différentes selon ses goûts : différentes ambiances sont envisageables pendant le voyage, ce qui peut être particulièrement agréable pour les longs voyages. Ces différentes fragrances peuvent également être très utiles pour masquer ou filtrer une odeur indésirable de tabac par exemple. Le passager peut également choisir de laisser le système de ventilation en marche sans pour autant être en présence d'une fragrance.

Une telle distribution des cavités, sous la forme de portions angulaires en anneau, permet de facilement augmenter le nombre de cavités sans pour autant devoir modifier le mécanisme d'actionnement associé. Le seul changement à effectuer pour augmenter le nombre de fragrances disponibles au passager réside dans la taille des cavités de la cartouche et dans la taille des ouvertures des plateaux tournants qui leur correspondent.

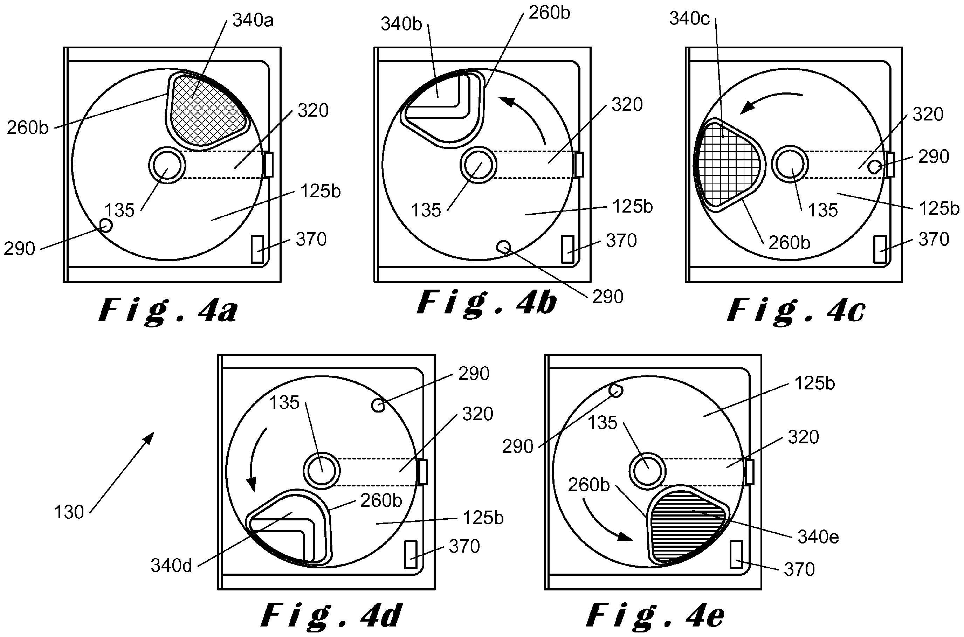

Les figures 4a à 4b illustrent une vue du haut du mécanisme permettant le changement de fragrances effectué par l'ensemble comprenant la cartouche 130 et le sélecteur 125a, 125b. La figure ne montre que le plateau tournant 125b situé sur la cartouche 130. Cependant, la vue du bas serait représentée de la même manière. L'axe mobile 135 ainsi que la fente 320 de la cartouche 130 sont également visibles sur la figure.

Dans le mode de réalisation décrit à la figure 3, où la cartouche comprend cinq cavités dont trois sont dédiées à loger une fragrance et deux sont laissées vides, les figures 4a à 4b illustrent le passage de l'ouverture 260b de la plaque tournante 125b sur chacune des cinq positions. La figure 4a illustre la première position de la plaque tournante où la fragrance libérée provient de la cavité 340a de la figure 3. Pour passer à la deuxième position, illustrée sur la figure 4b, la plaque tournante va tourner dans le sens antihoraire d'un angle d'environ 60 degrés. Cette position correspond à une cavité vide de la cartouche correspondant à la cavité 340b de la figure 3. La figure 4c illustre l'effet d'une rotation supplémentaire dans le sens antihoraire d'environ 60 degrés par rapport à la deuxième position permettant de libérer la deuxième fragrance logée dans la cavité 340c. De la même manière, les figures 4d et 4e illustrent l'ouverture du plateau tournant située respectivement sur les cavités 340d pour le quatrième position et 340e pour la cinquième position de la cartouche.

Préférentiellement, pour passer à une position ultérieure, la plaque tournante va tourner dans le sens horaire. En effet, il y a une zone par laquelle la plaque tournante ne passe pas pendant son utilisation. Cette zone se trouve au-dessus de la fente 320, ou entre les cavités 340a et 340e.

L'alternance de cavités vides et de cavités logeant une fragrance permet au passager de régler l'intensité de la fragrance diffusée par un actionnement du sélecteur en va-et-vient. Dans le même but, il est également possible de placer l'ouverture du plateau tournant en une position intermédiaire entre la cavité vide et la cavité remplie. Lorsque l'ouverture recouvre en totalité l'ouverture de la cavité, alors la diffusion est totale, lorsque l'ouverture recouvre seulement une partie de l'ouverture de la cavité comportant l'agent volatil, alors la diffusion n'est que partielle.

La vitesse du plateau tournant pour passer d'une fragrance à une utilisation sans fragrance est avantageusement de 0.5 seconde. Par suite, la vitesse de transition pour passer de la première position à la cinquième position, et vice-versa, est de 2.5 secondes.

Ce changement rapide entre différentes positions est rendu possible grâce au mécanisme d'actionnement 150 illustré sur les figures 1 et 2, et déjà décrit en partie.

Dans un mode de réalisation préféré, le moteur 140 est de type moto-réducteur pas à pas. Ce type de moteur standard est choisi pour des raisons de coût et de disponibilité sur le marché. Comme illustré sur les figures 1 et 2, l'arbre et le pignon de transmission sont à portion angulaire. En effet, la plage de rotation des plateaux tournants n'est pas de 360°, mais de 240° comme discuté ci-dessus.

C'est la combinaison de l'arbre 220, du pignon de transmission 230 et du pignon droit 240 qui permet une démultiplication du mouvement induit par le moto-réducteur 140 de telle sorte que le sélecteur passe d'une position à la suivante en 0.5 secondes et de la première position à la cinquième position en 2.5 secondes, et vice-versa. La combinaison de ces trois éléments forme un organe de démultiplication qui permet d'obtenir de telles performances avec un moteur standard.

Un système de commande du dispositif pourra comprendre un module de programmation et l'électronique associée afin de permettre au passager de contrôler le pulseur et le moteur du mécanisme d'actionnement électrique. Préférentiellement, le passager peut contrôler le distributeur de fragrances à partir d'une commande au volant.

Le système de commande permet au conducteur de mettre le distributeur de fragrances sous tension, de choisir l'intensité de la fragrance diffusée, de choisir le débit du flux d'air diffusé et/ou de choisir la fragrance. Avantageusement, le système de commande indique lorsqu'une fragrance de la cartouche est vide.

Dans un premier temps, le système de commande permet au passager de choisir la fragrance qu'il souhaite diffuser. Ensuite, il peut également régler le débit du flux d'air généré par le pulseur. Le système de commande permet également au passager de régler l'intensité de la fragrance qu'il souhaite diffuser.

Le système de commande pourra en outre permettre au passager de préprogrammer une séquence de diffusion qu'il souhaite en choisissant une durée de diffusion, une fragrance et son intensité. L'intensité pourra également varier au cours du temps ; il est dès lors possible de commencer par une diffusion totale qui devient partielle au cours du temps. Le système de commande permet également de choisir une fréquence de diffusion. Par exemple, le passager peut choisir de diffuser une certaine fragrance pendant une durée choisie, et puis ne rien diffuser pendant une autre durée.

Le système de verrouillage de la cartouche va désormais être décrit. Le système de verrouillage conforme à l'invention comprend un organe mobile qui correspond ici au plateau 125b décrit précédemment. Comme déjà indiqué, le plateau 125b ouvre et ferme les ouvertures de la cartouche amovible tel que décrit ci-dessus.

Comme illustré sur les figures 1 et 2, dans un premier mode de réalisation, le système comprend en outre un déclencheur se présentant dans ce premier mode sous la forme d'un bouton 250 de verrouillage/déverrouillage destiné à autoriser une extraction de ladite cartouche hors du boîtier en cas d'actionnement, ceci dans un premier mode d'utilisation du dispositif. Cependant, l'actionnement du déclencheur, ici du bouton, est bloqué dans un second mode d'utilisation de manière à empêcher à la cartouche de sortir du boîtier.

Le premier mode d'utilisation du dispositif concerne une utilisation normale du dispositif de diffusion par un passager. Le second mode d'utilisation se réfère par exemple à l'utilisation du véhicule pendant la période précédant la livraison du véhicule.

Sur la face visible 170a de la seconde partie du boîtier 170, une lumière permet l'insertion du bouton 250 de verrouillage/déverrouillage. Ce bouton est préférentiellement accessible au passager et est fixé sur la partie visible du boîtier lorsque ce dernier est inséré dans le véhicule automobile, en particulier dans la boîte à gants ou sur la planche de bord. Le bouton 250 est ici situé au-dessus de la façade 310 de la cartouche.

Une partie du système de verrouillage situé à l'intérieur du boîtier 170 est illustrée de manière plus détaillée sur la figure 2. Une plaque 255 servant d'interface avec les éléments qui vont suivre est fixée sur une partie du bouton interne au boîtier. Sur cette plaque, une équerre 260 est prévue. Elle est dimensionnée pour coopérer avec le bord du plateau 125b. Une tige 265 placée au même niveau que le bouton est également fixée sur la plaque 255. La tige s'étend selon une direction d'actionnement du bouton. La tige, dont la longueur correspond sensiblement à la longueur de la cartouche, comprend à son extrémité une rotule 295 à laquelle est relié un ergot 270.

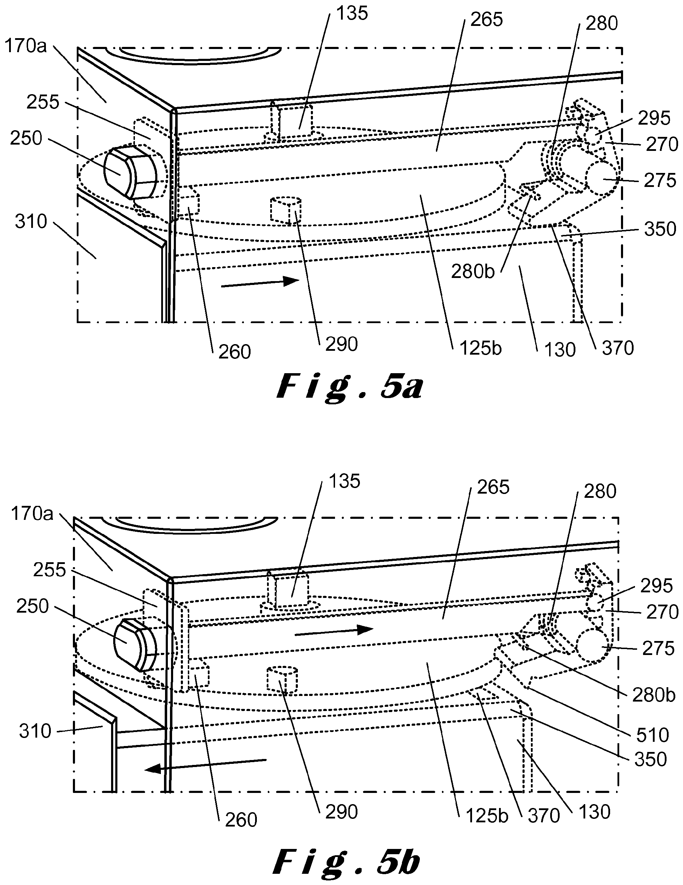

L'ergot 270 peut tourner autour d'un axe d'articulation s'étendant dans une direction transversale à celle de l'actionnement de la tige. Cependant, un ressort de rappel 280 dont la partie centrale est fixée sur l'ergot 270 tend à repositionner l'ergot dans une position de verrouillage de la cartouche en absence d'un actionnement du bouton 250. L'ergot possède une forme en L dont le coin comprend un pignon d'articulation 275. Le ressort de rappel 280 entoure le pignon 275. Une des tiges du ressort 280a est posée sur une face arrière 360 de la cartouche, et la seconde tige 280b est posée sur une face supérieure d'une base du L de l'ergot, la seconde tige étant visible sur les figures 5a et 5b. L'ergot est articulé sur le boîtier, ici sur la seconde partie 170a dudit boîtier.

La tige 265, l'axe de rotation 295 et le ressort de rappel 280 forment des moyens de transmission reliant cinématiquement le bouton d'actionnement 250 à l'ergot 270.

Une face inférieure de la base de l'ergot comprend un crochet 510 visible sur la figure 5b. D'autre part, le couvercle de la cartouche 130 comprend une cavité ou encoche 370 située sous l'ergot lorsque la cartouche est insérée dans le boîtier. L'encoche 370 est visible sur la figure 3. Les dimensions de l'encoche 370 correspondent aux dimensions du crochet 510, de telle sorte que le crochet puisse se loger dans l'encoche 370 de la cartouche.

Lorsque la cartouche 130 est insérée dans le boîtier, le crochet 510 s'engage dans l'encoche 370 tel qu'illustré sur la figure 5a. La forme du crochet est telle que lorsqu'il pénètre dans l'encoche de la cartouche, la cartouche est bloquée à l'intérieur du boîtier.

L'actionnement du bouton 250 libère la cartouche par l'intermédiaire des moyens de transmission, tel qu'illustré sur la figure 5b. La tige 265 suit le mouvement du bouton et va pousser la partie supérieure de l'ergot 270 par l'intermédiaire de la rotule 295. Ledit ergot 270 pivote alors autour du pignon 275. Le dos du L de l'ergot 270 se redresse et la base de l'ergot va également pivoter. Le crochet 510, placé à l'extrémité de la base du L va de cette manière sortir de l'encoche 370. Un ressort situé derrière la cartouche, non visible sur les figures, pourra pousser la cartouche en dehors du boîtier de manière à faciliter son extraction. Autrement dit, l'actionnement du bouton permet l'extraction de la cartouche en dehors du boîtier.

Comme déjà dit, le système de verrouillage décrit ci-dessus ne fonctionne cependant que dans le premier mode d'utilisation du dispositif. Dans le second mode d'utilisation de la présente invention, le bouton d'actionnement est bloqué afin d'empêcher une extraction de la cartouche du boîtier du dispositif de diffusion même en cas de tentative d'actionnement du bouton.

Pour cela, dans un mode de réalisation préférentiel de la présente invention, le plateau 125b, comprend sur sa face supérieure une butée de blocage empêchant l'actionnement du déclencheur, ici du bouton. La butée de blocage est ici fixée sur la face supérieure du plateau 125b près du bord. Elle a la forme d'un pion 290. Elle opère un blocage du bouton selon une direction transversale à un rayon dudit plateau 125b.

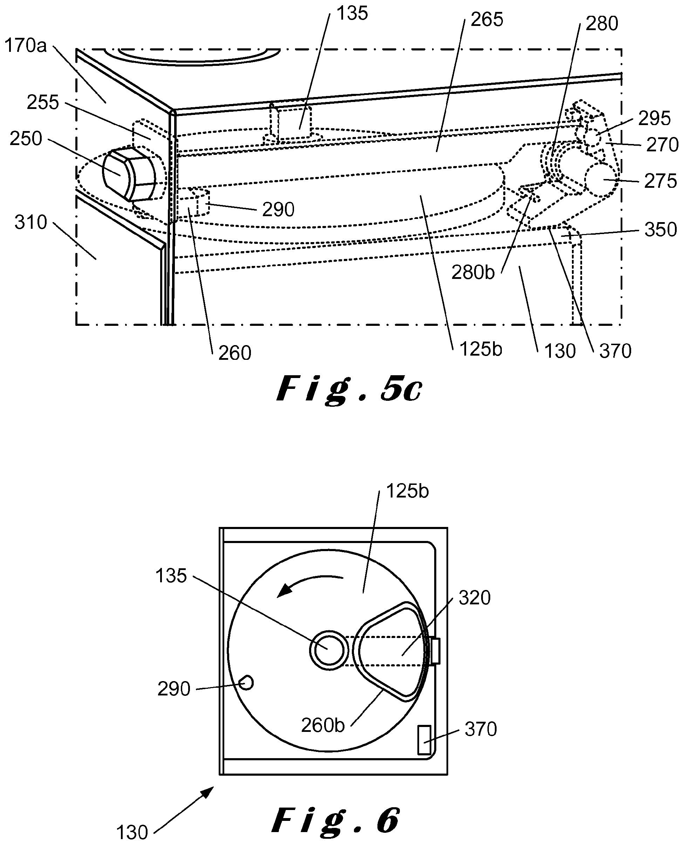

Lorsque le plateau 125b est mis dans une position dans laquelle la butée de blocage 290 se trouve en vis-à-vis de l'équerre 260 tel qu'illustré sur la figure 5c, l'actionnement du bouton 250 est bloqué et l'extraction de la cartouche amovible du boîtier est empêchée.

Si l'on se rapporte à nouveau aux figures 4a à 4e, on constate que, dans la première plage de déplacement correspondant à la course du plateau entre les positions de la figure 4a à celle de la figure 4e, et inversement, l'actionnement du bouton 250 est libre car la butée de blocage 290 ne bloque pas l'actionnement du bouton. Cependant, lorsque le plateau 125b tournant se trouve au-dessus de la fente 320, ou dans une seconde plage de déplacement du plateau, tel qu'illustré sur la figure 6, la butée de blocage se trouve en vis-à-vis de l'équerre et l'actionnement du bouton est bloqué.

Des moyens de commande du plateau, correspondant au système de commande décrit précédemment, sont avantageusement configurés de telle sorte que le plateau se déplace dans la première plage de déplacement du système dans le premier mode d'utilisation et dans la seconde plage de déplacement du système dans le second mode d'utilisation.

Les différentes plages de déplacement du plateau sont préprogrammées dans le système de commande. Avant la livraison du véhicule, le système de commande ne va autoriser que la seconde plage de déplacement afin d'empêcher une extraction de la cartouche et l'utilisation du dispositif. Après la livraison du véhicule, la première plage de déplacement sera choisie de manière à permettre une utilisation du dispositif de diffusion et afin de déverrouiller le bouton pour permettre à l'utilisateur de changer de cartouche.

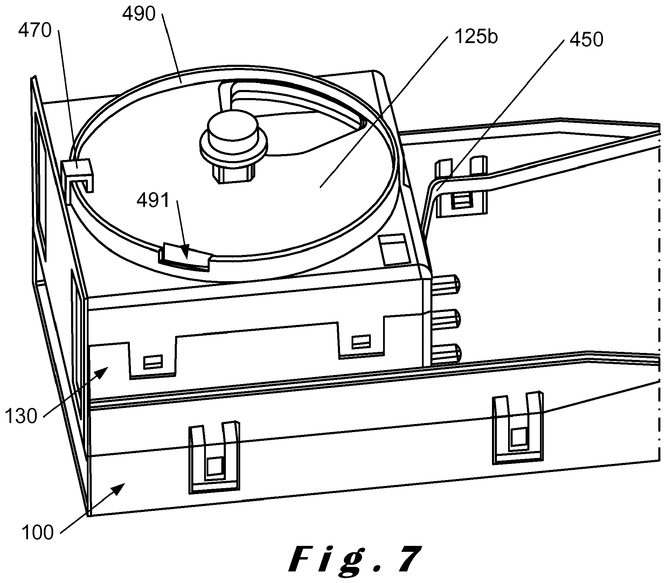

Comme illustré à la figure 7, selon une variante de réalisation, ledit déclencheur est un ressort 450, fixe par rapport au boîtier 100. Ledit ressort 450 est configuré pour agir sur ladite cartouche amovible 130. Ledit ressort est ici un ressort à lame dont la partie en contact avec ladite cartouche 130 n'est pas visible. Lorsque la cartouche 130 n'est pas présente, le ressort 150 est détendu. Lors de l'introduction de la cartouche 130, le ressort 150 est mis en tension. Il est alors susceptible d'entrainer l'expulsion de la cartouche 130, pour autant qu'un déplacement de cette dernière, selon sa direction d'introduction/extraction, ne soit pas bloqué.

Un tel blocage pourra être réalisé grâce à ladite butée de blocage qui prend ici la forme d'un bord périphérique 490, angulairement interrompue, dudit plateau 125b. De manière complémentaire, ladite cartouche amovible 130 comprend, par exemple, un ergot 470 configuré pour coopérer avec ledit bord périphérique 490 du plateau 125b. Ladite butée 490 opère ici un blocage radial.

En fonction du positionnement angulaire du plateau 125b, ledit ergot 470 est situé soit en vis-à-vis dudit bord périphérique 490, et la cartouche est alors bloquée par le plateau 125b, malgré l'action du ressort 450, soit ledit ergot 470 est situé en regard d'une encoche 491 formée sur un secteur angulaire dudit bord périphérique 490, et la cartouche est alors libre de se déplacer selon sa direction d'introduction/extraction, en particulier sous l'effet du ressort 450. Ce dernier provoque alors une expulsion, de préférence partielle, de la cartouche 130 hors du boîtier 100. L'utilisateur peut alors se saisir de ladite cartouche 130.

En variante, il est à noter que le ressort 450 pourra être omis. Le déclencheur de l'invention est alors formé par la simple correspondance de position entre l'ergot 470 de la cartouche et l'encoche 491 du bord périphérique 490 du plateau 125b, l'utilisateur pouvant alors opérer l'extraction de ladite cartouche 130 en la saisissant par sa face frontale, notamment sa façade 310.

Il est à noter que, dans ce second mode de réalisation, la seconde plage de déplacement est formée par n'importe quelle portion ou n'importe quelle valeur de position angulaire du plateau 125b, autre que celle où l'encoche 291 est en vis-à-vis de l'ergot 470 de la cartouche 130. Il y alors recouvrement de la première et de la seconde plage de déplacement. L'emploi d'un bouton devient optionnel.

Le plateau 125b est avantageusement amené en position d'extraction de la cartouche 130 par lesdits moyens de commande évoqué plus haut.

Bien que le dispositif de verrouillage conforme à l'invention ait été décrit dans le cadre d'un dispositif de diffusion de plusieurs fragrances, il pourra aussi être utilisé avec des cartouches ne servant qu'à une seule fragrance. Il pourra aussi être utilisé avec des cartouches servant à une autre application qu'un système de diffusion.

## Revendications

1. Système de verrouillage d'une cartouche amovible (130) dans un boîtier (100), ledit système comprenant un organe mobile, destiné à fermer et/ou ouvrir une et/ou des ouvertures (260a , 260b) de ladite cartouche (130), ledit système comprenant en outre un déclencheur (250, 450), destiné à autoriser une extraction de ladite cartouche (130) hors du boîtier (100) en cas d'actionnement, ledit organe mobile étant configuré pour bloquer l'actionnement dudit déclencheur (250, 450) de manière à empêcher à la cartouche (130) de sortir du boîtier (100) **caractérisé en ce que** ledit organe mobile est un plateau (125a, 125b) comprenant une butée de blocage (290, 490) empêchant l'actionnement du déclencheur, ledit déclencheur étant un bouton de verrouillage/déverrouillage (250) de ladite cartouche et ladite butée (290) est configurée pour empêcher l'actionnement du bouton (250), ledit système de verrouillage comprend en outre un ergot (270) relié au bouton (250) par des moyens de transmission.

2. Système selon la revendication précédente comprenant en outre des moyens de commande de l'organe mobile configurés pour que l'organe mobile se déplace dans la première plage de déplacement dans un premier mode d'utilisation du système et dans la seconde plage de déplacement dans un second mode d'utilisation du système.

3. Système selon l'une des revendications précédentes, dans lequel ledit plateau (125a, 125b) est mobile en rotation.

4. Système selon l'une des revendications précédentes, dans lequel lesdits moyens de transmission sont configurés pour que ledit ergot (270) se cale dans une cavité creusée sur la cartouche amovible (130) lorsque la cartouche (130) est insérée dans le boîtier (100) et se relâche lorsque le bouton (250) est actionné.

5. Système selon la revendication 3 dans lequel ledit déclencheur est un ressort (450) configuré pour agir sur ladite cartouche amovible (130) et ladite butée (490) est un bord périphérique, angulairement interrompue, dudit plateau (125a, 125b).

6. Système selon la revendication précédente dans lequel ledit bord périphérique du plateau (125a, 125b) est configuré pour coopérer avec un ergot (470) de ladite cartouche amovible (130).

7. Ensemble comprenant le système de verrouillage selon l'une quelconque des revendications précédentes et ladite cartouche amovible (130).

8. Ensemble selon la revendication 5 dans lequel ladite cartouche (130) comprend une pluralité d'ouvertures (260a, 260b) et ledit organe mobile est configuré pour sélectionner l'ouverture ouverte parmi les ouvertures (260a, 260b) de la cartouche (130).

9. Dispositif de diffusion d'agent volatil comprenant ledit ensemble selon l'une quelconque des revendications 5 ou 6.

10. Dispositif de diffusion d'agent volatil selon la revendication 9 dans lequel ladite cartouche amovible (130) est pourvue de cavités (340a, 340b, 340c, 340d, 340e) destinées à loger au moins un agent volatil.

## Patentansprüche

1. System zur Verriegelung einer entfernbaren Patrone (130) in einem Gehäuse (100), wobei das System ein bewegliches Organ enthält, das dazu bestimmt ist, eine und/oder mehrere Öffnungen (260a, 260b) der Patrone (130) zu schließen und/oder zu öffnen, wobei das System außerdem einen Auslöser (250, 450) enthält, der dazu bestimmt ist, im Fall einer Betätigung eine Entnahme der Patrone (130) aus dem Gehäuse (100) zu erlauben, wobei das bewegliche Organ konfiguriert ist, die Betätigung des Auslösers (250, 450) zu blockieren, um die Patrone (130) daran zu hindern, aus dem Gehäuse (100) auszutreten, **dadurch gekennzeichnet, dass** das bewegliche Organ eine Platte (125a, 125b) ist, die einen Blockieranschlag (290, 490) enthält, der die Betätigung des Auslösers verhindert, wobei der Auslöser ein Verriegelungs-/Entriegelungsknopf (250) der Patrone ist, und der Anschlag (290) konfiguriert ist, die Betätigung des Knopfes (250) zu verhindern, wobei das Verriegelungssystem außerdem einen Zapfen (270) enthält, der durch Übertragungseinrichtungen mit dem Knopf (250) verbunden ist.

2. System nach dem vorhergehenden Anspruch, das außerdem Steuereinrichtungen des beweglichen Organs enthält, die konfiguriert sind, damit das bewegliche Organ sich in einer ersten Verwendungsart des Systems im ersten Verschiebebereich und in einer zweiten Verwendungsart des Systems im zweiten Verschiebebereich verschiebt.

3. System nach einem der vorhergehenden Ansprüche, wobei die Platte (125a, 125b) drehbeweglich ist.

4. System nach einem der vorhergehenden Ansprüche, wobei die Übertragungseinrichtungen konfiguriert sind, damit der Zapfen (270) sich in einem auf der entfernbaren Patrone (130) ausgehöhlten Hohlraum verkeilt, wenn die Patrone (130) in das Gehäuse (100) eingeführt wird, und sich löst, wenn der Knopf (250) betätigt wird.

5. System nach Anspruch 3, wobei der Auslöser eine Feder (450) ist, die konfiguriert ist, auf die entfernbare Patrone (130) einzuwirken, und der Anschlag (490) ein winkelmäßig unterbrochener Umfangsrand der Platte (125a, 125b) ist.

6. System nach dem vorhergehenden Anspruch, wobei der Umfangsrand der Platte (125a, 125b) konfiguriert ist, mit einem Zapfen (470) der entfernbaren Patrone (130) zusammenzuwirken.

7. Einheit, die das Verriegelungssystem nach einem der vorhergehenden Ansprüche und die entfernbare Patrone (130) enthält.

8. Einheit nach Anspruch 5, wobei die Patrone (130) eine Vielzahl von Öffnungen (260a, 260b) enthält, und das bewegliche Organ konfiguriert ist, die offene Öffnung unter den Öffnungen (260a, 260b) der Patrone (130) auszuwählen.

9. Abgabevorrichtung einer flüchtigen Substanz, die die Einheit nach einem der Ansprüche 5 oder 6 enthält.

10. Abgabevorrichtung einer flüchtigen Substanz nach Anspruch 9, wobei die entfernbare Patrone (130) mit Hohlräumen (340a, 340b, 340c, 340d, 340e) versehen ist, die dazu bestimmt sind, mindestens eine flüchtige Substanz aufzunehmen.

## Claims

1. System for locking a removable cartridge (130) in a casing (100), said system having a movable member intended to close and/or open one and/or several openings (260a, 260b) in said cartridge (130), said system also having a release mechanism (250, 450) intended to permit removal of said cartridge (130) from the casing (100) when actuated, said movable member being designed to prevent actuation of said release mechanism (250, 450) such as to prevent removal of the cartridge (130) from the casing (100), **characterized in that** said movable member is a plate (125a, 125b) including a blocking stop (290, 490) preventing actuation of the release mechanism, said release mechanism being a locking/unlocking button (250) for said cartridge and said stop (290) being designed to prevent actuation of the button (250), said locking system also including a lug (270) connected to the button (250) by transmission means.

2. System according to the preceding claim, also including means for controlling the movable member, designed such that the movable member moves within the first range of movement in a first operating mode of the system and in the second range of movement in a second operating mode of the system.

3. System according to either of the preceding claims, in which said plate (125a, 125b) is movable in rotation.

4. System according to one of the preceding claims, in which said transmission means are designed such that said lug (270) becomes wedged in a cavity formed in the removable cartridge (130) when the cartridge (130) is inserted in the casing (100), and is released when the button (250) is actuated.

5. System according to Claim 3, in which said release mechanism is a spring (450) designed to act on said removable cartridge (130) and said stop (490) is an angularly discontinuous peripheral edge of said plate (125a, 125b).

6. System according to the preceding claims, in which said peripheral edge of the plate (125a, 125b) is designed to cooperate with a lug (470) of said removable cartridge (130).

7. Assembly including the locking system according to any one of the preceding claims and said removable cartridge (130).

8. Assembly according to Claim 5, in which said cartridge (130) has a plurality of openings (260a, 260b) and said movable member is designed to select the opening (260a, 260b) in the cartridge (130) that is open.

9. Device for diffusing a volatile agent including said assembly according to any one of Claims 5 or 6.

10. Device for diffusing a volatile agent according to Claim 9, in which said removable cartridge (130) has cavities (340a, 340b, 340c, 340d, 340e) intended to receive at least one volatile agent.
